# DEMANDE DE BREVET EUROPEEN

(11) **EP 1 591 101 A1**
(43) Date de publication de la demande: **02.11.2005**
(21) Numéro de dépôt: 05290813.4
(22) Date de dépôt: 13.04.2005
(51) Int. Cl.: A61K 7/047

(54) **Dissolvant gel sans acetone**

(30) Priorité: 20.04.2004 FR 0404160
(71) Demandeur: Durlin France, 24000 Bergerac (FR)
(72) Inventeur: Deswartvaegher, Alain, 24100 Bergerac (FR); Forestier, Bernard, 24520 Saint Nexans (FR); Miard, Sophie, 24520 Saint Germain et Mons (FR)
(74) Mandataire: Ahner, Francis

(57) **Abrégé**

Composition cosmétique ou dermatologique thixotrope sans acétone pour démaquiller les ongles présentant une structure de gel et comprenant:
a) un ou plusieurs agent(s) rhéologique(s) de type urée-méthane associé à un ou plusieurs émollient(s), et un ou plusieurs agent(s) épaississant(s) cellulosique(s), et
b) une phase organique constituée d'un ou plusieurs solvant(s) organique(s), susceptible de dissoudre un vernis à ongles.

## Description

La présente demande concerne une composition pour démaquiller les ongles, en particulier un dissolvant de vernis à ongles.

On utilise depuis quelques années des dissolvants de vernis à ongles sous forme épaissie telle qu'une crème, un gel ou une pâte. Ce type de présentation facilite la prise du produit hors de son conditionnement sans perte significative, limite la diffusion du produit à la zone locale de traitement (la surface de l'ongle) et permet d'utiliser des quantités suffisantes pour obtenir l'effet cosmétique recherché.

Par rapport aux dissolvants classiques sous forme de solutions organiques, ce type de présentation permet un étalement plus facile sur l'ongle, un dosage mieux contrôlé de la quantité nécessaire pour le démaquillage et d'obtenir, grâce à un temps de contact du produit sur l'ongle plus long, une meilleure efficacité au niveau du démaquillage.

Les dissolvants de vernis à ongles de l'art antérieur sous forme de gel, de crème ou de pâte, contiennent des agents épaississants et/ou des gélifiants et des solvants organiques susceptibles de dissoudre ou décomposer les polymères de revêtement des ongles couramment utilisés dans les vernis à ongles comme par exemple la nitrocellulose.

Le brevet US 4 804 486 décrit des dissolvants de vernis à ongles sous forme de gel ou de crème à base de solvants organiques tels que le propylène carbonate, le dihydro-2-(3H)furanine ou le certon et un agent gélifiant anionique du type polymère poly(acide acrylique) neutralisé par des substances alcalines.

La demande internationale WO 87/04921 divulgue des dissolvants sous forme de gel contenant de l'acétone comme solvant, de l'hydroxypropylcellulose comme gélifiant et un agent de conditionnement des ongles du type amine grasse polyoxyéthylénée.

Le brevet EP 0 841 059 décrit des dissolvants sous forme de gel contenant un organopolysiloxane de nature élastomérique au moins partiellement réticulé associé à une phase organique contenant au moins un solvant organique ou un mélange de solvants organiques cosmétiques, susceptibles de dissoudre un vernis à ongles et présentant des paramètres moyens de solubilité dD, dP et dH à 25°C de HANSEN qui satisfont aux trois conditions suivantes:
1. dD ≤ 20 (J/cm³)^{1/2},
2. dP ≤ 10 (J/cm³)^{1/2}, et
3. dD ≤ 20 (J/cm³)^{1/2}.

La demande internationale WO 02/45663 décrit des vernis à ongles contenant des urées-méthanes et des solvants à base de cétones.

Ces compositions conduisent le plus souvent, après démaquillage, à un dessèchement de l'ongle, à une délipidation de sa surface, à un blanchissement et à des pertes de brillance de l'ongle. De plus, aucun de ces dissolvants de vernis à ongles ne permet d'apporter sur l'ongle, de façon satisfaisante, après application un toucher doux naturel persistant.

La demanderesse a découvert de manière surprenante que l'association d'un ou plusieurs agent(s) rhéologique(s) de type urée-méthane avec un ou plusieurs émollient(s) et un ou plusieurs agents épaississant(s) cellulosiques(s), et une phase organique constituée d'un ou plusieurs solvant(s) organique(s) cosmétique(s), susceptible(s) de dissoudre un vernis à ongles pouvait constituer un remarquable agent démaquillant des ongles.

La demanderesse a découvert en effet qu'une telle association permettait d'obtenir des compositions de démaquillage des ongles sous forme thixotrope ne présentant pas tous les inconvénients énoncés ci-dessus et conférant aux ongles de façon inattendue, après démaquillage, un toucher doux persistant et une brillance naturelle.

On entend par "solvant organique cosmétique susceptible de dissoudre un vernis à ongles", tout solvant organique cosmétiquement acceptable et susceptible de dissoudre le ou les polymère(s), les résines et/ou le(s) plastifiant(s) formant le film déposé sur l'ongle. Ledit solvant doit être capable en particulier de dissoudre la nitrocellulose et les autres constituants du vernis à ongles.

Le ou les agent(s) rhéologique(s)thixotrope(s) de type urée-méthane selon l'invention peut être choisis parmi les dérivés décrits dans le brevet EP 0 006 252.

Un des dérivés d'urée-méthane de la composition de l'invention est par exemple celui commercialisé sous le nom BYK®-410. On peut aussi utiliser un mélange de ces agents.

Le ou les agents épaississant(s) cellulosique(s) selon l'invention peut être notamment un agent épaississant de type éthylcellulose N300 ou de type hydroxyéthylcellulose commercialisé sous le nom Klucel®M, un silicone volatile comme la diméticone qui permet d'obtenir une texture plus homogène et sans grumeaux. On peut également utiliser un mélange de ces épaississants.

De préférence, la phase organique susceptible de dissoudre un vernis à ongles présente des paramètres de solubilité de Hansen se trouvant dans la sphère de solubilité de la nitrocellulose.

Par "sphère de solubilité de la nitrocellulose", on entend le volume sphérique à l'intérieur duquel tous les solvants présents solubilisent la nitrocellulose. Les coordonnées de HANSEN des solvants étant repérées dans un système à 3 dimensions dans lequel:
- l'axe des abscisses représente la composante polaire (delta p),
- l'axe des ordonnées représente la composante liaison hydrogène (delta h),
- l'axe perpendiculaire aux 2 premiers la composante dispersion (delta d).

De préférence, la distance entre le solvant et le centre de la sphère de solubilité est inférieure à 11,5.

Le tableau 1 ci-après présente les paramètres de solubilité de HANSEN des solvants présents dans la composition selon l'invention.

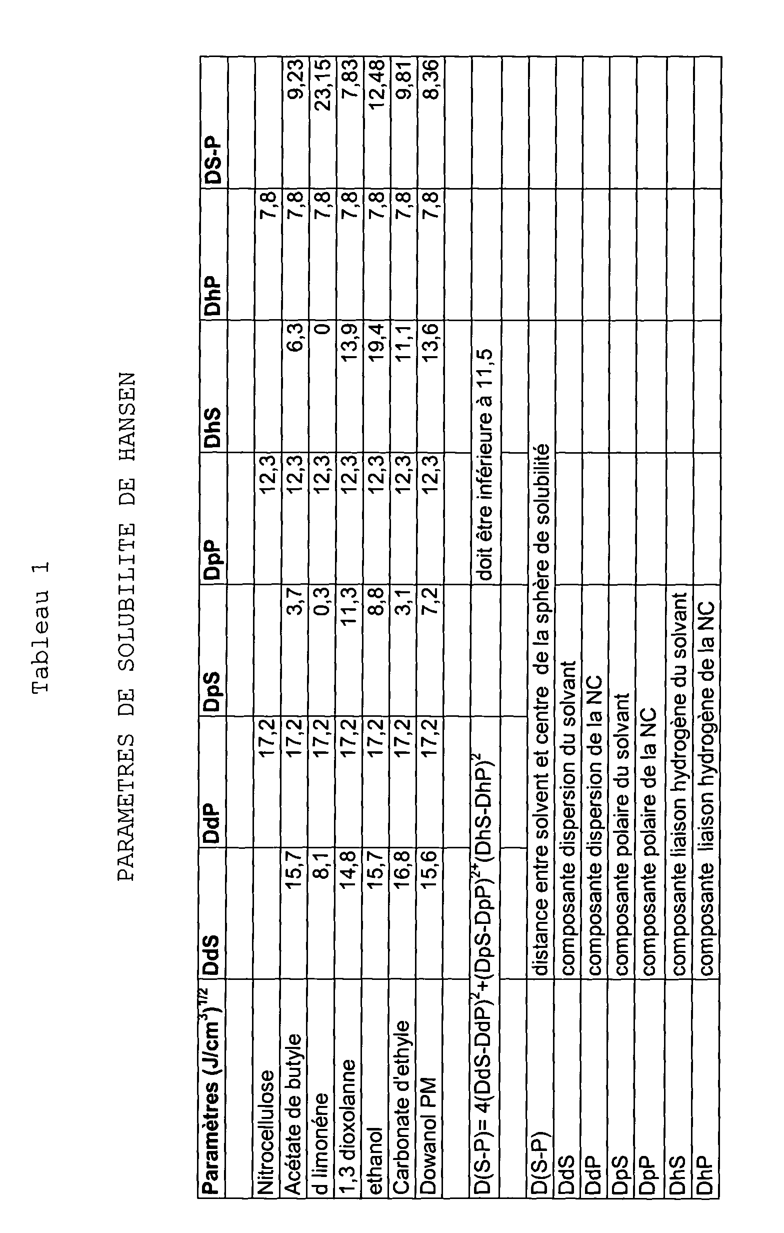

Selon l'invention, l'agent rhéologique est de préférence présent en une quantité comprise entre 0,1 à 1%, de préférence encore entre 0,5 et 0,7% en poids.

De préférence, la phase organique est constituée par un mélange d'au moins un solvant fortement polaire et d'au moins un solvant faiblement polaire. Le mélange peut également être constitué d'un mélange de solvants moyennement polaires.

Par "solvants moyennement polaires", on entend des solvants présentant un delta p des coordonnées de HANSSEN compris entre 4 et 7 (J/cm³)^{1/2}. L'homme du métier sait ce que signifie solvant fortement ou faiblement polaire.

De façon avantageuse, la phase organique comprend plusieurs solvants organiques cosmétiques dont le mélange répond aux conditions de polarité définie ci-dessus et qui sont choisis dans le groupe comprenant les esters acétiques (acétate d'éthyle, acétate de butyle), des esters carboniques (carbonate de méthyle, carbonate d'éthyle), les éthers de propylène glycol (Dowanol® PM) et les acétates (1,3-dioxolanne).

La phase organique peut comprendre également des solvants organiques ayant de faible capacité à dissoudre les vernis, comme par exemple des alcools isopropyliques ou butyliques ou éthyliques ou le limonène type D.

Parmi ces solvants, on utilise avantageusement les mélanges acétate de butyle/Dowanol®, acétate de butyle Dowanol®/éthanol, acétate de butyle/D limonène/carbonate d'éthyle.

Les émollients selon l'invention peuvent être choisis dans le groupe comprenant l'acétyl tributyl citrate, notamment Citroflex®A₄, le benzoate de saccharose, le dibutyl phthalate, le dioctyl phthalate, le tricresyl phtalate, le butyl phtalate, le dibutoxy éthyl phthalate, le diamylphthalate, le tosyl amide, N-éthyl-tosyl amide, l'acétate isobutyrate de saccharose, l'huile de camphre, l'huile de ricin, les citrate esters, les glyceryl diesters, les glyceryl triesters, le tributyl phosphate, le tri-phényl phosphate, le butyl glycolate, le benzyl benzoate, le butyl acétyl ricinoléate, le butyl stéarate, le dibutyl tartrate, et les adipates type dioctyladipate ou di-isobutyl adipate.

Avantageusement, l'émollient est choisi dans le groupe constitué par l'acétyl tributyl citrate, le benzoate de saccharose et l'acétate isobutyrate de saccharose.

De façon avantageuse, la phase organique est présente dans la phase gel de la composition à une concentration allant de 80 à 99% en poids, de préférence entre 95 et 97% en poids.

Le gel résultant de cette association peut être utilisé tel quel et constituer lui-même une composition pour le démaquillage des ongles. Il peut également être incorporé dans une formulation de dissolvant de vernis à ongles dans une quantité efficace pour obtenir à la fois la texture et la viscosité souhaitées et un bon démaquillage des ongles.

Les compositions selon l'invention se présentent de préférence sous forme de gels translucides ou transparents présentant une viscosité comprise entre 100 et 3000 mPa.s.

Elles peuvent contenir en plus des adjuvants classiques tels que des colorants, des parfums, des conservateurs, des filtres solaires et des agents hydratants. Ces adjuvants sont présents dans des quantités allant de préférence de 0 à 20% en poids par rapport au poids de la composition.

L'invention concerne également un procédé de démaquillage des ongles, caractérisé par le fait qu'on applique sur la surface de l'ongle une quantité efficace d'une composition constituée par ou contenant un gel tel que défini ci-dessus.

### Exemple: Préparation de gels.

Les gels dissolvants n° 1 à 10 sont préparés en mélangeant les ingrédients selon le tableau 2.

Les gels n°1 à 6 sans agent rhéologique de type urée-méthane et/ou agent épaississant cellulosique sont des exemples comparatifs.

On obtient des gels faciles à étaler en 7 à 10, conférant aux ongles après démaquillage et nettoyage avec un coton, un toucher doux naturel persistant. Les ongles démaquillés présentent un aspect naturel. Ils restent brillants et il n'apparaît pas après séchage du dissolvant, un film blanchâtre disgracieux à la surface des ongles et des cuticules.

Les gels 9 et 10 présentent de plus un aspect limpide.

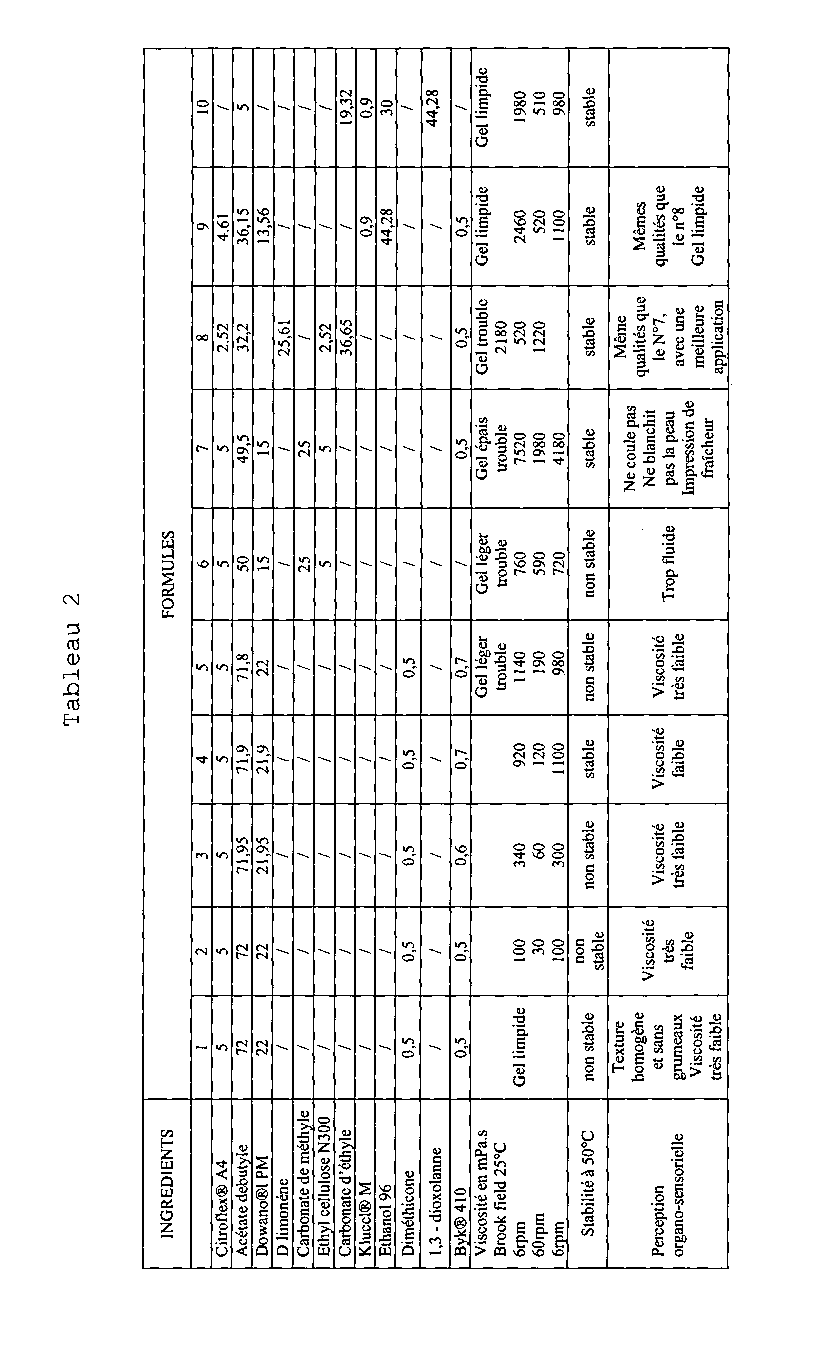

## Revendications

1. Composition cosmétique ou dermatologique thixotrope sans acétone pour démaquiller les ongles présentant une structure de gel et comprenant:
a) un ou plusieurs agent(s) rhéologique(s) de type urée-méthane associé à un ou plusieurs émollient(s), et un ou plusieurs agent(s) épaississant(s) cellulosique(s), et
b) une phase organique constituée d'un ou plusieurs solvant(s) organique(s), susceptible de dissoudre un vernis à ongles.

2. Composition selon la revendication 1, **caractérisée en ce que** la phase organique présente des paramètres de HANSEN se trouvant dans la sphère de solubilité de la nitrocellulose.

3. Composition selon la revendication 1, **caractérisée en ce que** le ou les agent(s) rhéologiques(s) est présent en une quantité comprise entre 0,1 à 1%, de préférence entre 0,5 et 0,7% en poids.

4. Composition selon la revendication 1, **caractérisée en ce que** la phase organique est constituée par un mélange d'au moins un solvant fortement polaire et d'au moins un solvant faiblement polaire.

5. Composition selon la revendication 1, **caractérisée en ce que** la phase organique est constituée par un mélange de solvants moyennement polaires.

6. Composition selon l'une quelconque des revendications 1 à 10, **caractérisée en ce que** les solvants sont choisis dans le groupe comprenant les esters acétiques, des esters carboniques, les éthers de propylène glycol, des alcools isopropyliques ou butyliques, ou des solvants ayant de faibles capacités à dissoudre les vernis, comme par exemple le limonène type D.

7. Composition selon l'une quelconque des revendications 1 à 6, **caractérisée en ce que** l'agent épaississant cellulosique est choisi dans la groupe comprenant l'éthylcellulose et l'hydroxypropyl cellulose.

8. Composition selon l'une quelconque des revendications 1 à 7, **caractérisée en ce que** l'agent rhéologique de type urée-méthane est le BYK 410®.

9. Composition selon l'une quelconque des revendications 1 à 8, **caractérisée en ce que** l'émollient est choisi dans le groupe comprenant l'acétyl tributyl citrate, le benzoate de saccharose, le dibutyl phthalate, le dioctyl phthalate, le tricresyl phtalate, le butyl phtalate, le dibutoxy éthyl phthalate, le diamylphthalate, le tosyl amide, N-éthyl-tosyl amide, l'acétate isobutyrate de saccharose, l'huile de camphre, l'huile de ricin, les citrate esters, les glyceryl diesters, les glyceryl triesters, le tributyl phosphate, le tri-phényl phosphate, le butyl glycolate, le benzyl benzoate, le butyl acétyl ricinoléate, le butyl stéarate, le dibutyl tartrate, et les adipates type dixityladipate ou di-isobutyl adipate.

10. Composition selon la revendication 9, **caractérisée en ce que** l'émollient est choisi dans le groupe constitué par l'acétyl tributyl citrate, le benzoate de saccharose et l'acétate isobutyrate de saccharose.

11. Procédé de démaquillage des ongles **caractérisé par le fait qu'**on applique sur la surface de l'ongle une quantité efficace d'une composition constituée par ou contenant un gel tel que défini selon les revendications 1 à 10.
